# EUROPEAN PATENT APPLICATION

(11) **EP 3 323 311 A1**
(43) Date of publication of application: **23.05.2018**
(21) Application number: 17202404.4
(22) Date of filing: 17.11.2017
(51) Int. Cl.: A45F 5/00, A61F 5/02, A41C 1/08, A45F 3/14, A45F 3/12

(54) **RUNNING ACCESSORY**

(30) Priority: 17.11.2016 IT 201600116446
(71) Applicant: PAVIS S.P.A., 20144 Milano (MI) (IT)
(72) Inventor: FRANGI, Barbara, 21026 Gavirate VA (IT)
(74) Representative: Spina, Alessandro

(57) **Abstract**

The invention relates to a running accessory (100) for the transport of water and food supplies, said running accessory comprising:
i) a lumbar orthopedic belt (110) made of an elastic fabric wherein a plurality of pockets (111, 112) are formed, said pockets (111, 112) housing respective flexible darts suitable to support the lumbar region and the lateral/abdominal region of a user;
ii) fastening means (113, 114) arranged at opposite ends of said lumbar orthopedic belt (110) in a horizontal direction (H), said fastening means being adapted to allow fixing of the lumbar belt around the waist of a user;
iii) one or more containers (120, 130) suitable for transporting objects. The containers (120, 130) are fixed on an external side of the lumbar orthopedic belt (110) at a rear portion thereof, which is intended to contact the lumbar region of a user.

## Description

### Technical field of the invention

The present invention generally relates to the field of accessories for runners and race walkers. More particularly, the invention relates to a running accessory comprising one or more containers for the transport of liquid and food supplies.

### Background

In the practice of running, both during training sessions and long-distance competitions such as half marathons and marathons, as well as during off-road training and the so-called "trail run" competitions through woods and the like, runners and race walkers typically carry a water supply, for example contained in one or more bottles, and a food supply, for example comprising energy bars and the like. These supplies are necessary for training and are even compulsory in official competitions on the road and off road.

Transport accessories for runners and race walkers are well known in the art and commercially available. Among these there are backpacks and belt bags provided with suitable containers where food, bottles and other objects or even documents can be stored. These accessories for runners and race walkers are typically equipped with fastening means such as snap-fits and/or tear-off members, as well as with pull straps allowing to adapt them to the body of a user in order to limit the oscillation of the containers during movement.

Among the transport accessories for runners and race walkers belt bags are particularly diffused, because they have a remarkably smaller size compared to backpacks. Known belt bags have containers arranged at a front side thereof, i.e. at the abdomen of a user in an operating condition. Other known belt bags have instead containers positioned at a rear side thereof, i.e. at the lumbar region of a user in an operating condition, thus exploiting a natural concavity of the body where their oscillation is generally less annoying to a user.

One of the most common problems of known belt bags is that during trainings or competitions the inevitable oscillations of the containers cause a progressive loosening of the pull straps over time, so that a runner must periodically intervene to adjust them. This problem is very annoying to enthusiasts and practitioners, because it can force them to one or more stops during a sport activity or a competition.

Also known are orthopedic belts configured to support the lumbar region and the abdominal region of a user when moving in order to provide a support during lifting efforts. A lumbar orthopedic belt of the aforementioned type is for example described in patent US 4099524.

Lumbar orthopedic belts for working activities are also known.

Patent US 5241704 discloses, for example, a lumbar orthopedic belt comprising a rear portion provided with stiffening darts and intended to be placed in contact with the lumbar area of the back of a user, and a front portion that is removably detachable to the rear portion, for example by way of pull members comprising of Velcro® elements. The front portion can be associated with a storage container comprising one or more pockets. In one of the embodiments described in the patent, the storage container forms with the front portion of the belt an apron that can be attached to the rear portion by way of braces.

Patent US 5470000 describes another lumbar orthopedic belt provided with a storage container similar to the previous one, but wherein the rear portion and the front portion are made as a single piece having hook and loop fastening means at the ends. A storage element can be attached to the belt at the front portion by using the hook and loop fastening means. The storage container is similar to a belt bag that can be restrained to the orthopedic belt.

A lumbar orthopedic belt with a removable storage container that is very similar to the previous one is described in patent US 5693006.

### Summary of the Invention

The technical problem underlying and solved by the present invention is therefore to provide a running accessory configured for the transport of water and food supplies that is suitable to overcome the drawbacks mentioned above with reference to the prior art. This problem is solved by a running accessory according to claim 1. Preferred features of the present invention are specified in the dependent claims.

The running accessory according to the invention comprises a lumbar orthopedic belt made of an elastic fabric wherein flexible support darts suitable to sustain the lumbar region and the lateral/abdominal area of a user's body are fitted. The running accessory also includes one or more storage containers restrained to the lumbar orthopedic belt at a rear portion thereof, i.e. the portion intended to contact the lumbar region of a user.

In other words, the running accessory according to the invention is a combination between a lumbar orthopedic belt comprising flexible support darts for the lumbar region and the lateral/abdominal region of a user's body and a plurality of containers suitable to store water and food supplies, wherein the containers are restrained on the rear portion of the belt.

Unlike prior art lumbar orthopedic belts comprising storage containers, the arrangement of the containers at the rear portion of the belt offers the advantage of stabilizing them when running thus substantially neutralizing their oscillations. This is clearly not achievable by known lumbar orthopedic belts, because they are provided with storage containers arranged at their front portions so as to facilitate access to users who need to have a plurality of tools available while operating substantially in a steady position.

Thanks to the elasticity of the fabric of which a lumbar orthopedic belt is typically made and to the presence of its flexible darts, the running accessory according to the invention perfectly adheres to the waist of a user and substantially assumes the same curvature of the back in the lumbar region. The flexible darts allow to keep the lumbar orthopedic belt fully extended, thus effectively avoiding deformations caused in operation due to the movements of a user, while sustaining his/her lumbar, lateral, and abdominal regions. Consequently, the containers attached to the rear portion of the lumbar orthopedic belt are much more stable against possible oscillations if compared to prior art lumbar belts for sports.

This is not possible with known belt bags, which, apart from allowing to tighten the pull straps, are rather stiff and poorly adjustable on the body of a user.

According to a preferred embodiment of the invention, the containers are anchored to the lumbar orthopedic belt at discrete pointa. This makes it possible to exploit the elasticity of the fabric of which the belt is made so as to maximize its wrapping effect around the user's waist, and thus to minimize the oscillations of the containers restrained thereto during the practice of sports.

The containers may be mounted on the lumbar orthopedic belt by way of seams, tearing elements, zippers or equivalent means.

According to an embodiment of the invention, the containers of the running accessory are arranged on a common supporting member mounted on the lumbar orthopedic belt by way of seams, hook and loop elements, zippers or equivalent anchoring means. This allows to achieve a better arrangement of the containers and to simplify their assembly on the lumbar orthopedic belt.

Further advantages, features and the operation mode of the present invention will become clear from the following detailed description of some embodiments thereof that are disclosed for exemplifying and non-limiting purposes.

### Brief description of the figures

Reference will be made to the figures of the accompanying drawings, in which:
- Figure 1 is a top view showing an outer side of a running accessory according to the invention;
- Figure 2 is a top view showing an inner side of a running accessory according to the invention;
- Figures 3 to 5 are a front view, a rear view, and a side view, respectively, showing the waist of a user wearing the running accessory shown in figures 1 and 2.

### Detailed description of preferred embodiments

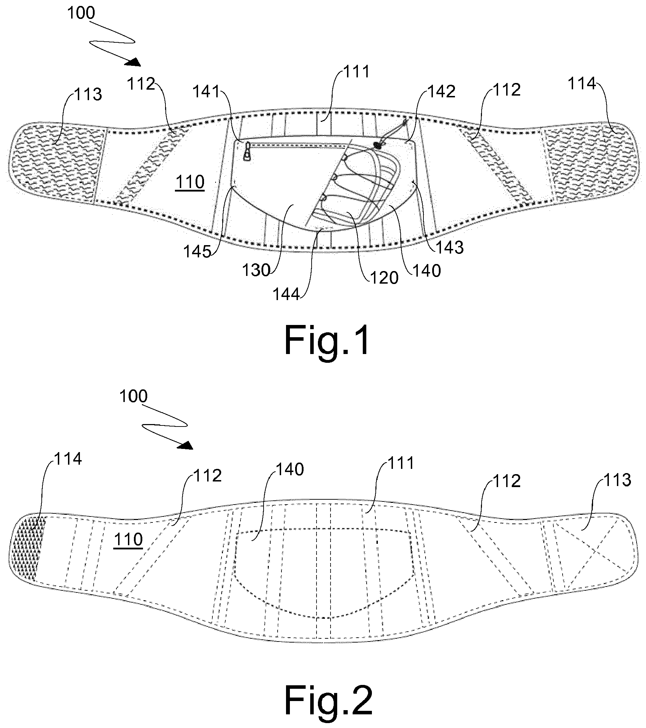

With particular reference to figures 1 and 2, a running accessory according to the invention is generally indicated by the reference numeral 100 and is shown lying on a plane on which it stretches out in a horizontal direction H and in a vertical direction V that are mutually perpendicular.

More particularly, figure 1 shows an external side of the running accessory 100, i.e. the side intended to be exposed and be accessible to a user during its use, while figure 2 shows an inner side of the running accessory 100, i.e. the side intended to contact the waist of a user during use.

The running accessory 100 comprises a lumbar orthopedic belt 110 made of an elastic fabric wherein a plurality of pockets 111 are formed, e.g. five pockets in the illustrated example. The pockets 111 accommodate respective flexible (not shown) darts intended to sustain the lumbar region of a user. With reference to an operating condition of the running accessory 100, it will be appreciated that the pockets 111 are formed in a rear portion of the lumbar orthopedic belt 110.

The lumbar orthopedic belt 110 also comprises a plurality of pockets 112, e.g. two in the illustrated example, adapted to accommodate respective (not shown) darts intended to sustain the lateral/abdominal region of a user. The pockets 112 are formed in a lateral/frontal portion of the belt.

The lumbar orthopedic belt 110 extends in the horizontal direction H, which corresponds to a winding direction around the wearer's waist. The flexible darts are arranged transversely to the longitudinal direction L. More particularly, the flexible darts that are housed in the pockets 111, and are intended to support the lumbar region of the user, are arranged substantially perpendicular to the horizontal direction H, while the flexible darts that are housed in the pockets 112, and are intended to support the lateral/abdominal region of the user, are inclined with respect to the horizontal direction H and converge in the vertical direction V.

At the opposite ends in the horizontal direction H, the lumbar orthopedic belt 110 includes fastening means allowing to fasten it around the waist of a user. In the illustrated example, the fastening means comprise a first pull member 113 and a pull member 114 that are complementary to each other and include, for example, a plurality of hooks elements and a plurality of loops elements, respectively, that are commercially known under the trademark Velcro®.

The size of the belt and the number and arrangement of the flexible darts are not limiting features of the invention.

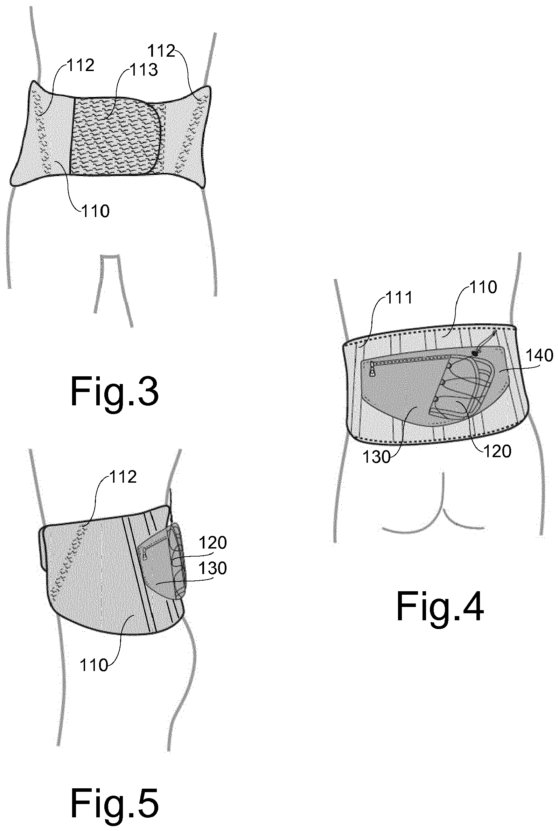

As shown in figure 3, a user wears the running accessory 100 by tightening the lumbar orthopedic belt 110 around the waist and fixing it at the abdomen by way of the fastening means 113, 114.

The running accessory 100 according to the invention further comprises one or more containers suitable for carrying objects, in particular water and/or food supplies. Such containers are provided with respective fastening means such as strings or zippers and are restrained to the outer side of the lumbar orthopedic belt 110 at its rear portion, i.e. the portion intended to contact the lumbar region of a user.

For instance the illustrated embodiment features a bottle-holder 120 provided with pull strings and one or more pockets 130 e.g. having a zipper. It will be appreciated that the container type is not a limiting feature of the invention.

The containers may be restrained to the lumbar orthopedic belt 110 by way of seams, hook and loop members, zippers or equivalent anchoring means.

According to a preferred embodiment of the invention, the containers are restrained to the lumbar orthopedic belt 110 at discrete points. This allows to exploit the elasticity of the fabric of which the lumbar orthopedic belt 110 is made in order to maximize its wrapping effect around the user's waist and thus to minimize the oscillations of the containers when running and, more generally, during the practice of a sport.

The discrete anchorage points of the containers are preferably located close to or at the pockets 111 of the flexible darts intended to support the lumbar region. This provides the advantage of allowing to exploit the spreading effect of the darts on the lumbar orthopedic belt 110 in order to minimize the oscillations of the containers.

The containers are preferably anchored in the middle of the rear portion of the lumbar orthopedic belt 110, for example symmetrically relative to the pockets 111 of the flexible darts.

In the embodiment of the invention shown in the figures, the containers of the running accessory, e.g. the bottle-holder 120 and the pockets 130 with the zippers, are advantageously disposed on a common supporting member 140, for example having a polygonal shape, that is in turn mounted on the lumbar orthopedic belt 110 by ways off seams, hook and loop members, zippers or equivalent anchoring means. This allows to better position the containers and to simplify their assembly on the lumbar orthopedic belt 110.

As it may be seen, the discrete anchoring or mounting points of the supporting member 140 on the lumbar orthopedic belt 110 are located e.g. at the vertices of the polygonal supporting member 140. These points, five in the example shown in the drawings, are respectively indicated by reference numbers 141, 142, 143, 144 and 145.

The present invention has hereinabove been disclosed with reference to preferred embodiments thereof. It will be appreciated that further embodiments may exists all of which are based on the same inventive idea, as defined by the scope of protection of the claims set out below.

## Claims

1. A running accessory (100) for the transport of water and food supplies, said running accessory comprising:
i) a lumbar orthopedic belt (110) made of an elastic fabric wherein a plurality of pockets (111, 112) are formed, said pockets (111, 112) housing respective flexible darts suitable to support the lumbar region and the lateral/abdominal region of a user;
ii) fastening means (113, 114) arranged at opposite ends of said lumbar orthopedic belt (110) in a horizontal direction (H), said fastening means being adapted to allow fixing of the lumbar belt around the waist of a user;
iii) one or more containers (120, 130) suitable for transporting objects, said containers (120, 130) being fixed on an external side of the lumbar orthopedic belt (110) at a rear portion thereof, which is intended to be arranged at the lumbar region of a user in an operating condition.

2. A running accessory (100) according to claim 1, wherein the containers (120, 130) are anchored to the lumbar orthopedic belt (110) at discrete anchoring points.

3. A running accessory (100) according to claim 2, wherein said anchoring discrete points of the containers (120, 130) are located close to or in correspondence with the pockets (111) housing the flexible darts intended to support the lumbar region of a user.

4. A running accessory (100) according to any one of claims 1 to 3, wherein the containers (120, 130) are anchored to the rear portion of the lumbar orthopedic belt (110) at a central portion thereof intended to support the lumbar region of a user, and wherein the containers are arranged symmetrically relative to the pockets (111) housing the flexible darts.

5. A running accessory (100) according to any one of claims 1 to 4, wherein the containers (120, 130) are arranged on a common supporting member (140), in turn fixed to the rear portion of the lumbar orthopedic belt (110).

6. A running accessory (100) according to any one of claims 1 to 5, wherein the containers (120, 130) are fixed to the lumbar orthopedic belt (110) by way of seams, hook and loop members, zippers or equivalent anchoring means.

7. A running accessory (100) according to any one of claims 1 to 6, wherein the containers comprise at least one bottle-holder (120) and at least one pocket (130) having a zipper.
